(19) Europäisches Patentamt / European Patent Office / Office européen des brevets

(11) **EP 4 585 152 B1**

(12) **EUROPEAN PATENT SPECIFICATION**

(45) Date of publication and mention
of the grant of the patent:
**03.06.2026 Bulletin 2026/23**

(21) Application number: **24151101.3**

(22) Date of filing: **10.01.2024**

(51) International Patent Classification (IPC):
***A61B 6/00*** *(2024.01)*

(52) Cooperative Patent Classification (CPC):
**A61B 6/545; A61B 6/4441**

(54) **AUTOMATICALLY DETERMINING AN OPERATION MODE FOR AN X-RAY IMAGING SYSTEM**

AUTOMATISCHE BESTIMMUNG EINES BETRIEBSMODUS FÜR EIN RÖNTGENBILDGEBUNGSSYSTEM

DÉTERMINATION AUTOMATIQUE D'UN MODE DE FONCTIONNEMENT POUR UN SYSTÈME D'IMAGERIE À RAYONS X

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(43) Date of publication of application:
**16.07.2025 Bulletin 2025/29**

(73) Proprietor: **Siemens Healthineers AG**
**91301 Forchheim (DE)**

(72) Inventors:
• **Bernhardt, Philipp**
**91301 Forchheim (DE)**
• **Ravi, Arpitha**
**91058 Erlangen (DE)**

(74) Representative: **Siemens Healthineers Patent Attorneys**
**Postfach 22 16 34**
**80506 München (DE)**

(56) References cited:
**US-A1- 2011 311 030     US-A1- 2021 386 389**
**US-A1- 2023 020 252     US-A1- 2023 298 736**

**Description**

The present invention is directed to a computer implemented method for automatically determining an operation mode for an X-ray imaging system, which comprises an X-ray source and an X-ray detector mounted on a C-arm. The invention is also directed to a method for X-ray imaging using an X-ray imaging system comprising said computer implemented method and to a computer implemented training method for training a machine learning model for classification for use in said computer implemented method. The invention is further directed to a corresponding data processing apparatus, an X-ray imaging system comprising said data processing apparatus, and to corresponding computer program products.

In X-ray medical imaging it is important that the X-ray imaging system is configured properly for the requested task. For example, a static situation such as bone images of legs or arms allows for long exposure times, whereas imaging of the lung requires considering motion blurring. Also, within certain disciplines, such as cardiac imaging of the coronary vessels, selecting a certain parametrization of the X-ray imaging system may be beneficial, since each projection or task within a diagnostic or interventional procedure has specific requirements.

In order to assist a user in achieving the optimum system condition, users may have the option to choose a comprehensive package of parameter lists, also denoted as organ programs, OGPs, or operating modes, which are settings specifically tailored to accomplish a given task. However, for various reasons, users may select a generic OGP, which is applicable for various use cases and therefore necessarily represents a compromise between the requirements of different use cases. Consequently, the selected OGP may not be optimal for the actual use case, which leads to a reduced performance of the X-ray imaging system and/or a reduced quality of the resulting X-ray images.

ResNet is a popular architecture for artificial neural networks, ANN, in particular convolutional neural networks, CNN, which was introduced in the publication K. He et al.: "Deep Residual Learning for Image Recognition", Proceedings of the IEEE Conference on Computer Vision and Pattern Recognition (CVPR), 2016, pp. 770-778. ResNet models are widely used for deep learning tasks due to their ability to enable the training of very deep neural networks. The ResNet-50 is a ResNet with a depth of 50 layers.

Using neural networks for supporting imaging operation of an imaging apparatus is known, e.g. from US 2023/0020252 A1.

It is an objective of the present invention to provide a possibility to automatically determine an operation mode for an X-ray imaging system as described above.

This objective is achieved by the subject matter of the independent claims. Further implementations and preferred embodiments are subject matter of the dependent claims.

The invention is based on the idea to treat the selection of an operation mode as a classification problem. A trained machine learning model, MLM, is used to carry out the classification depending on the angulation state of the C-arm and at least one X-ray image taken according to said angulation state.

According to an aspect of the invention, a computer implemented method for automatically determining an operation mode for an X-ray imaging system is provided. The X-ray imaging system comprises an X-ray source mounted on a C-arm of the X-ray imaging system and an X-ray detector mounted on the C-arm. Angulation data defining an angulation state of the C-arm is received. At least one X-ray image depicting an object according to the angulation state is received. The operation mode for the X-ray imaging system is selected as one of two or more predefined operation modes by applying a trained machine learning model, MLM, for classification to input data, the input data comprising the at least one X-ray image and the angulation data.

Unless stated otherwise, all steps of the computer-implemented method may be performed by a data processing apparatus, which comprises at least one computing unit. In particular, the at least one computing unit is configured or adapted to perform the steps of the computer-implemented method. For this purpose, the at least one computing unit may for example store a computer program comprising instructions which, when executed by the at least one computing unit, cause the at least one computing unit to execute the computer-implemented method.

In case the at least one computing unit comprises two or more computing units, certain steps carried out by the at least one computing unit may be understood such that different computing units carry out different steps or different parts of a step. In particular, it is not required that each computing unit carries out the steps completely. In other words, carrying out the steps may be distributed amongst the two or more computing units.

In general terms, a trained MLM may mimic cognitive functions that humans associate with other human minds. In particular, by training based on training data the MLM may be able to adapt to new circumstances and to detect and extrapolate patterns. Another term for a trained MLM is "trained function".

In general, parameters of an MLM can be adapted or updated by means of training. In particular, supervised training, semi-supervised training, unsupervised training, reinforcement learning, and/or active learning can be used. Furthermore, representation learning, also denoted as feature learning, can be used. In particular, the parameters of the MLMs can be adapted iteratively by several steps of training. In particular, within the training a certain loss function, also denoted as cost function, can be minimized. In particular, within the training of an ANN, the backpropagation algorithm can be used.

In particular, an MLM can comprise an ANN, a support vector machine, a decision tree and/or a Bayesian network, and/or the machine learning model can be based on k-means clustering, Q-learning, genetic algorithms, and/or association rules. In particular, an ANN can be or comprise a deep neural network, a convolutional neural network, or a convolutional deep neural network. Furthermore, an ANN can be an adversarial network, a deep adversarial network, and/or a generative adversarial network.

An operation mode defines, in particular, at least one predefined setting for the X-ray imaging system. The at least one setting may comprise one or more operating parameters for the X-ray imaging system, in particular the X-ray source, filters in the path of radiation, the X-ray detector, and so forth. The at least one setting may also comprise one or more parameters for image processing based on the data generated by the X-ray detector. The at least one setting may also comprise a respective specification whether or not certain functions are to be activated during the data acquisition or for the image processing and/or whether or not certain functions are to be activated during the data acquisition or for the image processing. An operation mode may for example correspond to a predefined organ program, OGP.

The X-ray imaging system may be configured according to the selected operation mode and one or more further X-ray images depicting the object may be generated by the x-ray imaging system configured accordingly. The at least one X-ray image may also be generated by the X-ray imaging system using, for example, a preliminary operation mode or preliminary settings, respectively.

In particular in the context of X-ray-based imaging of vessels, for example cardiac imaging or imaging of the brain, a contrast agent may be used for generating the one or more further X-ray images. In this case, the at least one X-ray image may for example be generated before the contrast agent is applied.

The angulation state corresponds, in particular, to the position of the X-ray source and the X-ray detector relative to the object or, in other words, to the direction of a straight line connecting the X-ray source to the X-ray detector, in particular its center, in three dimensions. Commonly, the angulation state of the C-arm is given by two rotation angles. One of them is denoted as orbital rotation angle or cranial / caudal angle, respectively. The other one is denoted as angular rotation angle or left anterior oblique / right anterior oblique angle, respectively.

The invention is based on the finding that, while neither the at least one X-ray image, in particular without applying a contrast agent, nor the angulation state alone provide sufficient information to reliably predict the intended imaging procedure for generating the further X-ray images. The combination of the at least one X-ray image and the angulation state, however, has been found to allow for a very reliable prediction. Therefore, according to the invention, the MLM uses both for the classification task.

According to several implementations, an information message informing a user about the selected operation mode is generated.

The information message may be output visually by a display and/or as a speech output, for example. The user can then initiate the configuration of the X-ray imaging system according to the selected operation mode or manually configure the X-ray imaging system accordingly.

According to several implementations, the X-ray imaging system is automatically configured according to the selected operation mode.

In particular, one or more control signals may be generated depending on the selected operation mode and the X-ray imaging system may be automatically configured depending on the one or more control signals.

In such implementations, the user does not have to carry out or initiate the configuration manually, which leads to a further increased reliability that the optimal operation mode is used and saves time for the user.

According to several implementations, configuring the X-ray imaging system according to the selected operation mode comprises setting a value of an X-ray exposure time assigned to the selected operation mode and/or a value of a frame rate assigned to the selected operation mode and/or at least one image processing parameter assigned to the selected operation mode and/or activating or deactivating a function of the X-ray imaging system assigned to the selected operation mode.

The function may for example be a function for averaging two or more of the further X-ray images for avoiding motion artifacts or another function for image processing, in particular.

Consequently, it is ensured that the further X-ray images are generated with optimal quality for the actual use case.

According to several implementations, the MLM comprises one or more artificial neural networks, ANNs, or one or more ANN modules of an ANN. The ANN or the ANN modules may, in particular, be a convolutional neural network, CNN, or CNN modules, respectively.

According to several implementations, image features are generated by applying a first feature extraction module, which is for example an ANN module, of the MLM to the at least one X-ray image. The operation mode is selected depending on the image features.

The first feature extraction module may be given or be based on a known ANN architecture for extracting features from images. In particular, the first feature extraction module may comprise a CNN, for example a residual neural network, ResNet, in particular a ResNet-50. However, also other types of ANN families, such as VGG, Inception, and so forth may be used.

According to several implementations, angulation features are generated by applying a second feature extraction module, which is for example an ANN module, of the MLM to the angulation data. The operation mode is selected depending on the angulation features.

The second feature extraction module may be given or be based on a known ANN architecture for extracting features, for example a multi-layer perceptron, MLP. Since the angulation data are given by relatively few numerical values, the second feature extraction module is, in particular, not designed as a CNN.

According to several implementations, the operation mode is selected depending on the image features and the angulation features.

Consequently, the MLM comprises two distinct feature extraction modules for extracting the relevant features from the two components of the input data, namely the at least one X-ray image and the angulation data, respectively. This approach has been found to predict the correct class for the operation mode very reliably.

According to several implementations, fused features are generated by fusing the image features with the angulation features by the MLM. The operation mode is selected depending on the fused features.

The fusion of the features may for example be carried out by concatenating the image features and the angulation features. In this way, the MLM, for example a further ANN module of the MLM, can use all the extracted features in a comprehensive manner to carry out the classification task.

According to several implementations, the operation mode is selected by applying at least one fully connected neural network layer of the MLM to the fused features.

Consequently, only a relatively low amount of memory resources and computational resources are required for the final classification.

According to several implementations, the angulation data comprises the angular rotation angle and the orbital rotation angle of the C-arm. In particular, the angulation data consists of the angular rotation angle and the orbital rotation angle.

Therefore, the complexity of the angulation data is particularly low and the angulation data is very easy to retrieve during the prediction phase of the MLM as well as during the training phase.

According to several implementations, the two or more predefined operation modes comprise a first operation mode for imaging right coronary arteries and/or a second operation mode for imaging left coronary arteries and/or a third operation mode for imaging a left ventricle.

These types of X-ray imaging are particularly sensitive to the settings defined by the operation modes and therefore benefit particularly significantly from the invention.

For use cases or use situations which may arise in a computer implemented method or another method according to the invention and which are not explicitly described herein, it may be provided that, in accordance with the method, an error message and/or a prompt for user feedback is output and/or a default setting and/or a predetermined initial state is set.

According to a further aspect of the invention, a method for X-ray imaging using an X-ray imaging system, which comprises an X-ray source and an X-ray detector mounted on a C-arm is provided. Therein, angulation data defining an angulation state of the C-arm is determined. The X-ray imaging system is configured, for example manually or automatically, according to at least one preliminary setting. At least one X-ray image depicting an object according to the angulation state and according to the at least one preliminary setting is generated by using the X-ray source and the X-ray detector. A computer implemented method for automatically determining an operation mode for the X-ray imaging system according to the invention is carried out, for example by at least one computing unit of the X-ray imaging system. The X-ray imaging system is configured, for example manually or automatically, according to the selected operation mode. A further X-ray image, in particular according to the angulation state, is generated according to the selected operation mode, in particular after configuring the X-ray imaging system according to the selected operation mode, by using the X-ray source and the X-ray detector.

For example, a contrast agent may be applied to the object after the at least one X-ray image is generated and before the further X-ray image is generated or while the further X-ray image is generated. Therefore, the total amount of contrast agent applied is not increased for generating the at least one X-ray image. Nevertheless, the combination of the at least one X-ray image with the angulation data still allows for a reliable prediction of the optimal operation mode.

According to a further aspect of the invention, a computer implemented training method for training an MLM for classification for use in a computer implemented method for automatically determining an operation mode for an X-ray imaging system according to the invention is provided. Therein, angulation training data defining a training angulation state of the C-arm is received. At least one training image depicting an object according to the training angulation state is received. A ground truth annotation for the angulation training data and the at least one training image is received. A predicted operation mode for the X-ray imaging system is selected as one of two or more predefined operation modes by applying the MLM to input training data, which comprises the at least one X-ray training image and the training angulation data. The MLM is updated depending on the predicted operation mode and the ground truth annotation, in particular using supervised training.

The described steps are, in particular, understood as a single training run. A plurality of such runs may be carried out consecutively, until a predetermined termination or convergence criterion regarding the second loss function is reached. Each set of at least one training image may be denoted as a training sample. The number of training samples may lie in the order of 100 or several times 100, for example 400 to 1000. The number of training epochs may for example lie in the order 100-1000. The total number of training runs is for example given by the product of the number of training samples and the number of training epochs.

A training image of the at least one training image may be an X-ray image or a digitally reconstructed radiograph, DRR, or another simulated X-ray image.

The ground truth annotation is the one of the two or more predefined operation modes, which the MLM shall predict if it is fully trained. The ground truth annotations may be manually generated by clinical experts such as image quality specialists, radiological technologists, and/or medical physicists with several years of experience in the field.

Unless stated otherwise, all steps of the computer-implemented method may be performed by a further data processing apparatus, which comprises at least one further computing unit. In particular, the at least one further computing unit is configured or adapted to perform the steps of the computer implemented training method. For this purpose, the at least one further computing unit may for example store a further computer program comprising instructions which, when executed by the at least one further computing unit, cause the at least one further computing unit to execute the computer implemented training method.

In case the at least one further computing unit comprises two or more computing units, certain steps carried out by the at least one further computing unit may be understood such that different further computing units carry out different steps or different parts of a step. In particular, it is not required that each further computing unit carries out the steps completely. In other words, carrying out the steps may be distributed amongst the two or more further computing units.

According to several implementations of the computer implemented training method, training image features are generated by applying a pre-trained first feature extraction module of the MLM to the at least one X-ray training image. Training angulation features are generated by applying a pre-trained second feature extraction module of the MLM to the angulation training data. Training fused features are generated by fusing the training image features with the training angulation features. The predicted operation mode is selected by applying at least one fully connected neural network layer of the MLM to the fused features. Network parameters, in particular weighting factors and bias factors, of the at least one fully connected neural network layer are updated depending on the predicted operation mode and the ground truth annotation. Applying the at least one fully connected neural network layer may for example also include applying a Softmax function as a last step.

In particular, a loss function, in particular a classification loss function, such as a cross-entropy loss function, is evaluated depending on the output of the at least one fully connected neural network layer and the ground truth annotation. The network parameters of the at least one fully connected neural network layer are updated depending on the value of the loss function, in particular by using the backpropagation algorithm.

The first feature extraction module as well as the second feature extraction module are pre-trained in such implementations. In other words, they are frozen for training the at least one fully connected neural network layer and their respective network parameters are not updated depending on the value of the loss function. Thus, the first feature extraction module and/or the second feature extraction module may be used for other purposes apart from the computer implemented method for automatically determining an operation mode for an X-ray imaging system according to the invention as well.

In alternative implementations, the first feature extraction module and the second feature extraction module are not pre-trained and are trained together with the at least one fully connected neural network layer based on the loss function as described.

According to several implementations of the computer implemented method for automatically determining an operation mode for an X-ray imaging system, the MLM is trained by using the computer implemented training method according to the invention.

In other words, carrying out the computer implemented method comprises carrying out the computer implemented training method in such implementations.

In alternative implementations, the steps for carrying out the computer implemented training method are not part of the computer implemented method according to the invention.

According to a further aspect of the invention, a data processing apparatus comprising at least one computing unit, which is adapted to carry out a computer implemented method according to the invention, is provided.

A computing unit may in particular be understood as a data processing device, which comprises processing circuitry. The computing unit can therefore in particular process data to perform computing operations. This may also include operations to perform indexed accesses to a data structure, for example a look-up table, LUT.

In particular, the computing unit may include one or more computers, one or more microcontrollers, and/or one or more integrated circuits, for example, one or more application-specific integrated circuits, ASIC, one or more field-programmable gate arrays, FPGA, and/or one or more systems on a chip, SoC. The computing unit may also include one or more processors, for example one or more microprocessors, one or more central processing units, CPU, one or more graphics processing units, GPU, and/or one or more signal processors, in particular one or more digital signal processors, DSP. The computing unit may also include a physical or a virtual cluster of computers or other of said units.

In various embodiments, the computing unit includes one or more hardware and/or software interfaces and/or one or more memory units.

A memory unit may be implemented as a volatile data memory, for example a dynamic random access memory, DRAM, or a static random access memory, SRAM, or as a non-volatile data memory, for example a read-only memory, ROM, a programmable read-only memory, PROM, an erasable programmable read-only memory, EPROM, an electrically erasable programmable read-only memory, EEPROM, a flash memory or flash EEPROM, a ferroelectric random access memory, FRAM, a magnetoresistive random access memory, MRAM, or a phase-change random access memory, PCRAM.

According to a further aspect of the invention, a further data processing apparatus comprising at least one further computing unit, which is adapted to carry out a computer implemented training method according to the invention, is provided.

According to a further aspect of the invention, an X-ray imaging system is provided. The X-ray imaging system comprises a C-arm, an X-ray source mounted on the C-arm and an X-ray detector mounted on the C-arm. The X-ray imaging system comprises at least one computing unit, which is adapted to carry out a computer implemented method for automatically determining an operation mode for the X-ray imaging system according to the invention. In other words, the X-ray imaging system comprises a data processing apparatus according to the invention.

According to a further aspect of the invention, a first computer program comprising first instructions is provided.

When the first instructions are executed by a data processing apparatus, the first instructions cause the data processing apparatus to carry out a computer implemented method for automatically determining an operation mode for the X-ray imaging system according to the invention.

According to a further aspect of the invention, a second computer program comprising second instructions is provided. When the second instructions are executed by a data processing apparatus, the second instructions cause the data processing apparatus to carry out a computer implemented training method according to the invention.

According to a further aspect of the invention, a third computer program comprising third instructions is provided. When the third instructions are executed by an X-ray imaging system according to the invention, in particular by the at least one computing unit of the X-ray imaging system, the third instructions cause the X-ray imaging system to carry out a method for X-ray imaging according to the invention.

According to a further aspect of the invention, a computer-readable storage medium storing a first computer program and/or a second computer program and/or a third computer program according to the invention is provided.

The first, second and third computer program and the computer-readable storage medium are respective computer program products comprising the first, second, and/or third instructions.

The first, second, and/or third instructions may be provided as program code, for example. The program code can for example be provided as binary code or assembler and/or as source code of a programming language, for example C, and/or as program script, for example Python.

Above and in the following, the solution according to the invention is described with respect to the claimed systems as well as with respect to the claimed methods. Features, advantages, or alternative embodiments herein can be assigned to the other claimed objects and vice versa. In other words, claims and embodiments for the systems can be improved with features described or claimed in the context of the respective methods. In this case, the functional features of the method are implemented by physical units of the system.

Furthermore, above and in the following, the solution according to the invention is described with respect to methods and systems for automatically determining an operation mode for an X-ray imaging system and for X-ray imaging as well as with respect to methods and systems for training an MLM. Features, advantages, or alternative embodiments herein can be assigned to the other claimed objects and vice versa. In other words, claims and embodiments for training the MLM can be improved with features described or claimed in the context of automatically determining an operation mode for an X-ray imaging system and X-ray imaging. In particular, datasets used in the methods and systems can have the same properties and features as the corresponding datasets used in the methods and systems for providing a trained MLM, and the trained MLMs provided by the respective methods and systems can be used in the methods and systems for automatically determining an operation mode for an X-ray imaging system and for X-ray imaging.

Further features and feature combinations of the invention are obtained from the figures and their description as well as the claims. In particular, further implementations of the invention may not necessarily contain all features of one of the claims. Further implementations of the invention may comprise features or combinations of features, which are not recited in the claims.

In the following, the invention will be explained in detail with reference to specific exemplary implementations and respective schematic drawings. In the drawings, identical or functionally identical elements may be denoted by the same reference signs. The description of identical or functionally identical elements is not necessarily repeated with respect to different figures.

In the figures,

FIG 1    shows schematically an exemplary implementation of an X-ray imaging system according to the invention;

FIG 2    shows a schematic flow diagram of an exemplary implementation of a method for X-ray imaging according to the invention;

FIG 3    shows a schematic flow diagram of an exemplary implementation of a computer implemented training method according to the invention;

FIG 4    shows a schematic block diagram of a first feature extraction module of an MLM for use in an exemplary implementation of a computer implemented method according to the invention during training of the first feature extraction module;

FIG 5    shows a schematic block diagram of a second feature extraction module of an MLM for use in a further exemplary implementation of a computer implemented method according to the invention during training of the second feature extraction module;

FIG 6    shows a schematic block diagram of a trained MLM for use in a further exemplary implementation of a computer implemented method according to the invention;

FIG 7    shows a diagram depicting angulation states of a C-arm for different X-ray imaging use cases;

FIG 8    shows schematically a multi-layer perceptron; and

FIG 9    shows schematically a convolutional neural network.

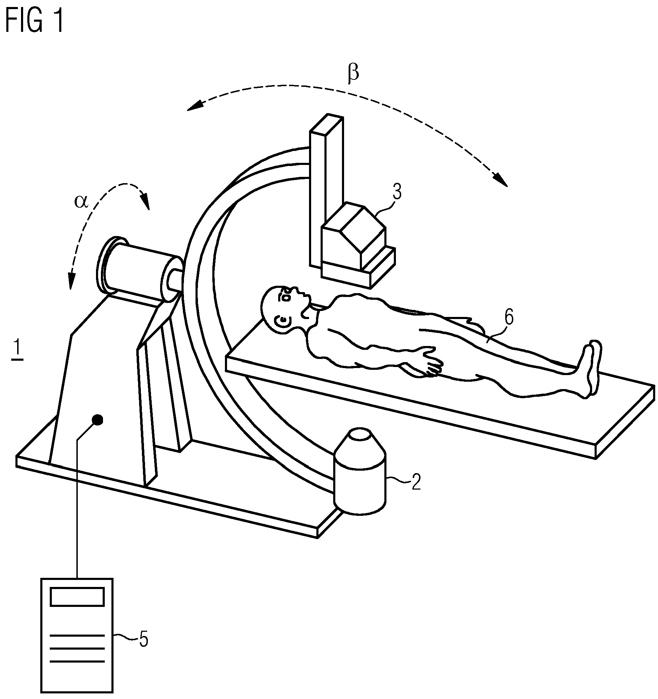

FIG 1 shows schematically an exemplary implementation of an X-ray imaging system 1 according to the invention. The X-ray imaging system 1 comprises a C-arm 4 with an X-ray source 2 and an X-ray detector 3 mounted on the C-arm 4. The C-arm 4 is designed such that the X-ray source 2 and an X-ray detector 3 can be rotated about at least two rotation axes corresponding to an angular rotation angle $\alpha$ and an orbital rotation angle $\beta$, respectively. This allows to adjust the C-arm 4 to different angulation states given by $(\alpha, \beta)$.

The X-ray imaging system 1 comprises at least one computing unit 5, which is adapted to carry out a computer implemented method for automatically determining an operation mode for the X-ray imaging system 1 according to the invention.

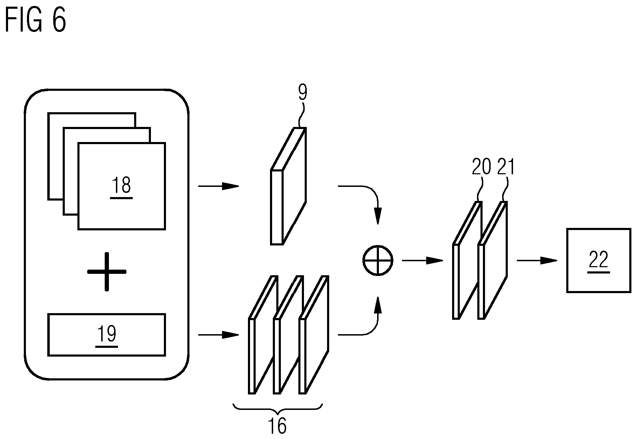

According to said computer implemented method, angulation data 19 (see FIG 6) defining the angulation state of the C-arm 4 is received and at least one X-ray image 18 (see FIG 6) depicting an object 6 according to the angulation state is received. The operation mode 22 (see FIG 6) for the X-ray imaging system 1 is selected as one of two or more predefined operation modes by applying a trained MLM for classification to input data, the input data comprising the at least one X-ray image 18 and the angulation data 19.

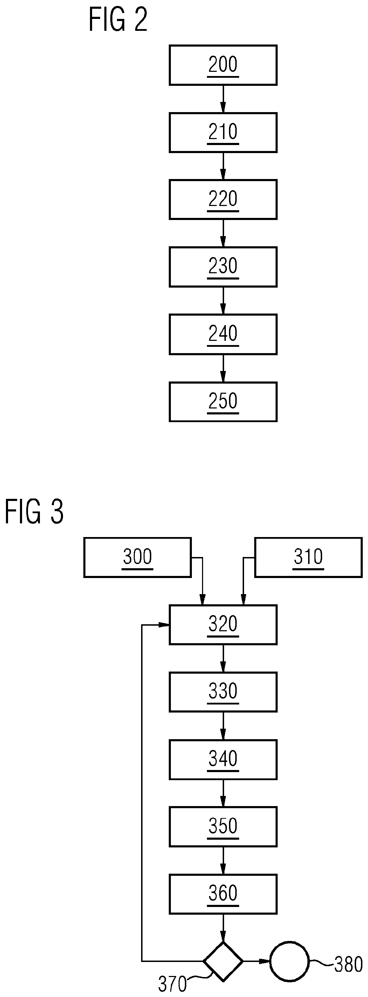

In particular, a method for X-ray imaging according to the invention may be carried out by the X-ray imaging system 1. A schematic flow diagram of such a method is shown in FIG 2.

In step 200, the angulation data 19 is received and in step 210, the X-ray imaging system 1 is configured according to at least one preliminary setting. In step 220, the at least one X-ray image 18 is received and in step 230, the trained MLM is applied to the input data to select the operation mode 22. In step 240, the X-ray imaging system 1 is configured according to the selected operation mode 22. After that, a further X-ray image is generated according to the selected operation mode 22 by using the X-ray source 2 and the X-ray detector 3 in step 250.

FIG 6 shows a schematic block diagram of the trained MLM for an exemplary implementation of a computer implemented method according to the invention. The MLM is designed as an ANN in this implementation. The MLM comprises a first feature extraction module 9, for example a CNN, in particular a ResNet-50, which is applied to the at least one X-ray image 18 to generate image features. The MLM comprises a second feature extraction module 16, for example an MLP, which is applied to the angulation data 19 to generate angulation features. The image features and the angulation features are fused, in particular concatenated. The operation mode 22 is selected by applying at least one fully connected neural network layer 20, 21 of the MLM to the fused features.

FIG 3 shows a schematic flow diagram of an exemplary implementation of a computer implemented training method for training the MLM according to the invention.

In step 300, the pre-trained first feature extraction module 9 is received and in step 310, the pre-trained second feature extraction module 16 is received. The parameters of both feature extraction modules 9, 16 are frozen for training the MLM further, in particular for training the at least one fully connected neural network layer 20, 21.

In step 320, angulation training data defining a training angulation state of the C-arm 4 is received and in step 330, at least one training image depicting an object 6 according to the angulation state is received. In step 340, a ground truth annotation for the angulation training data and the at least one training image is received.

In step 350, a predicted operation mode for the X-ray imaging system 1 is selected as one of two or more predefined operation modes by applying the MLM to input training data, which comprises the at least one X-ray training image and the training angulation data. In particular, training image features are generated by applying the pre-trained first feature extraction module 9 to the at least one X-ray training image and training angulation features are generated by applying the pre-trained second feature extraction module 16 to the angulation training data. Training fused features are generated by fusing the training image features with the training angulation features. The predicted operation mode is selected by applying the at least one fully connected neural network layer 20, 21 to the fused features.

In step 360, the MLM is updated depending on the predicted operation mode and the ground truth annotation. In particular, network parameters of the at least one fully connected neural network layer 20, 21 are updated depending on the predicted operation mode and the ground truth annotation.

In step 370, it is checked whether a predefined termination or convergence criterion is fulfilled. If this is the case, the trained MLM is provided in step 380. Otherwise, another training run is carried out including steps 320 to 330, for example with a new set of X-ray training images, angulation training data, and a corresponding ground truth annotation.

By means of the invention, the mere utilization of image datasets is extended by integrating the angulation data of the C-arm 4. This integration is significant since the angulation state of the C-arm 4 is a key factor in medical diagnostic processes. Clinicians routinely adjust this angulation state, for example to acquire diverse perspectives of the coronary artery, achieving differential magnification levels in radiographic imaging, and so forth.

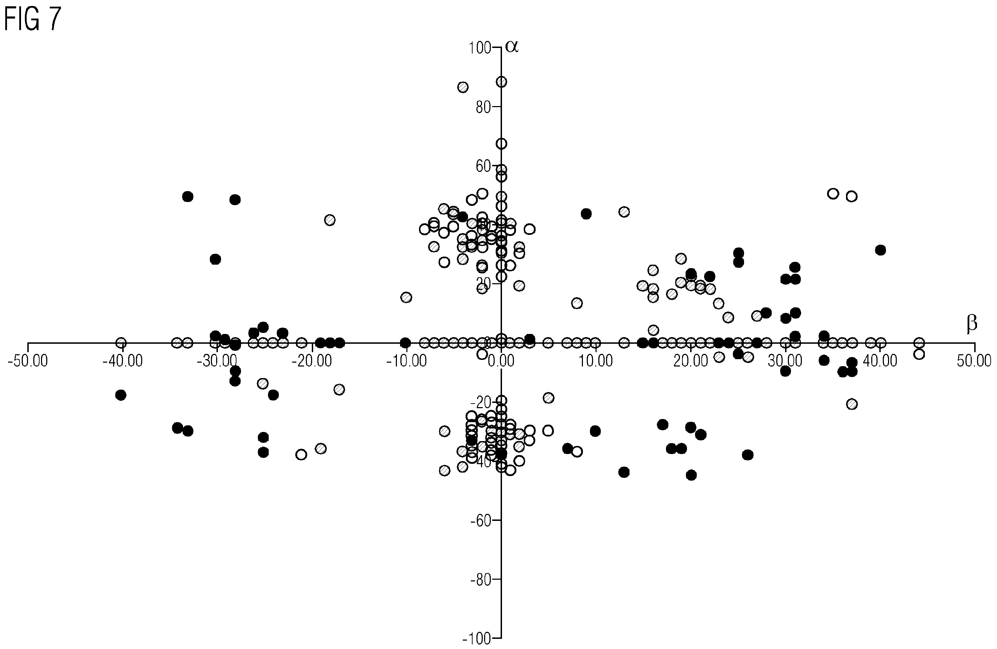

FIG 7 shows a diagram, which depicts the distribution of the angular rotation angle $\alpha$ and the orbital rotation angle $\beta$ for multiple use cases. The black filled circles correspond to left coronary artery, LCA, imaging, the shaded circles correspond to right coronary artery, RCA, imaging and the empty circles correspond to left ventricle, LV imaging. As can be seen from the diagram, is it not promising to try to classify the different imaging use cases based on the angulation alone. The invention overcomes this problem by combining the X-ray images with the angulation data as input for the classification.

The angulations may be systematically categorized as left anterior oblique, LAO, and right anterior oblique, RAO, for lateral views according to the angular rotation angle $\alpha$, and caudal and cranial for vertical orientations according to the orbital rotation angle $\beta$. Figure 3b provides a graphical representation of these orientations. The angulation data may be directly obtained from the C-arm 4 controller without requiring further pre-processing. This approach enhances the classification performance significantly by incorporating a broader spectrum of diagnostic information.

In several implementations, the least one training image are pre-processed X-ray images. The pre-processing may include a log transform, for example. Therein, an image is read and a natural logarithm is applied to the image values. The logarithm compresses the displayed brightness at the bright end and expands the dark end. Ultimately, all contrasts are harmonized in the whole image without depending any longer on the background. Alternatively or in addition, the pre-processing may include a square root transform. Therein, the image is read and the square root of the image values is computed. The focus of this is to harmonize the noise in the whole image, which originally follows the counting statistics in the raw image. This helps when performing a filtering task, for example. Alternatively or in addition, the pre-processing may include a scatter correction. Therein, the image is read and a scatter offset value is subtracted from it. To obtain this offset, one may first consider a portion of the collimator in the image. The mean value of this collimator region is calculated and represents the offset. If the log transform is also used, the logarithm is for example applied to the scatter corrected image.

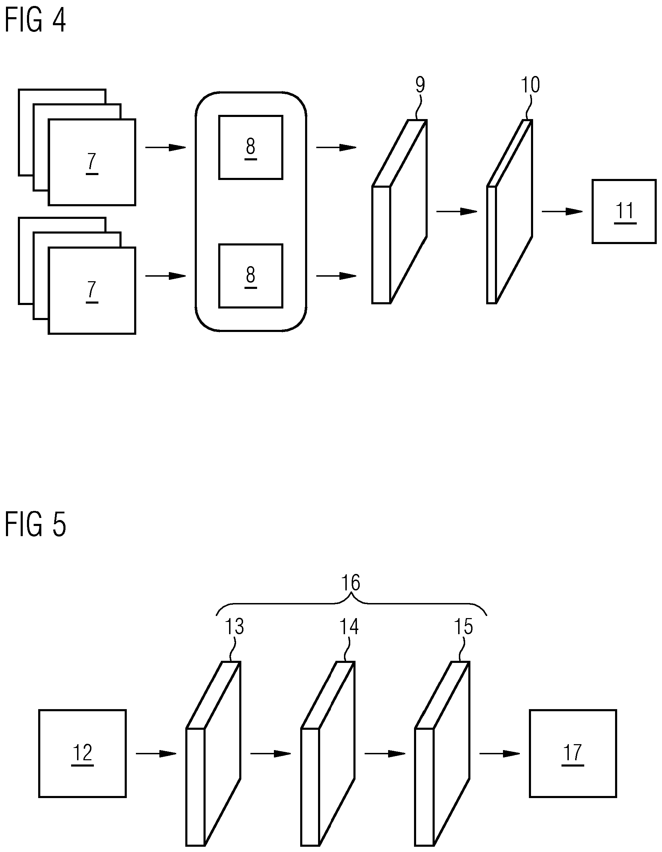

In several implementations, a pre-trained Resnet-50 architecture is employed for the first feature extraction module 9. This Resnet-50 model is for example pre-trained on the ImageNet dataset. Further training images 7 or pre-processed further training images 8 are provided as input to the Resnet-50 for feature extraction, and the extracted features are then passed through a fully connected layer 10 for classification, as depicted schematically in FIG 4.

For example, a batch size of 2 and a learning rate of $10^{-4}$ may be used as hyperparameters for training the first feature extraction module 9. For multi-class classification, a categorical cross entropy with a Softmax activation function may be used as the loss function, which may be optimized by using an Adam optimizer. Early stopping may be used with a patience value of 25 while monitoring the validation loss to avoid overfitting.

In several implementations, an MLP, for example consisting of three layers 13, 14, 15 is used for the second feature extraction module 16. The first two layers 13, 14 are used for feature extraction from further angulation training data 12, and the final layer 15 is used for classification, as depicted schematically in FIG 5.

For the training of the second feature extraction module 16, one may use a batch size of 100 and a learning rate of 0.01. The categorical-cross entropy with a Softmax activation may be used as a loss function and the Adam optimizer may also be used here. Also early stopping with a patience value of 25, while monitoring the validation loss may be used.

The full MLM may thus leverage the principles of transfer learning. The pre-trained feature extraction modules 9, 16 are integrated as shown in FIG. 6. Then the at least one fully connected neural network layer 20, 21 is trained as described above. Note that the fully connected layer 10 is then not required anymore. Specifically, when training the at least one fully connected neural network layer 20, 21, the Resnet-50 layers are frozen, maintaining the pre-existing weights acquired from the image data model, rendering these layers non-trainable. In a parallel manner, the MLP layers are similarly frozen, and loaded with pre-trained weights from the angulation data model. The resultant feature vectors from both models are concatenated and then fed into the at least one fully connected neural network layer 20, 21. This process culminates in a final fully connected layer 21, which is dedicated to the task of classification. For example, the same hyperparameters and loss function as used for training the first feature extraction module 9 may be used.

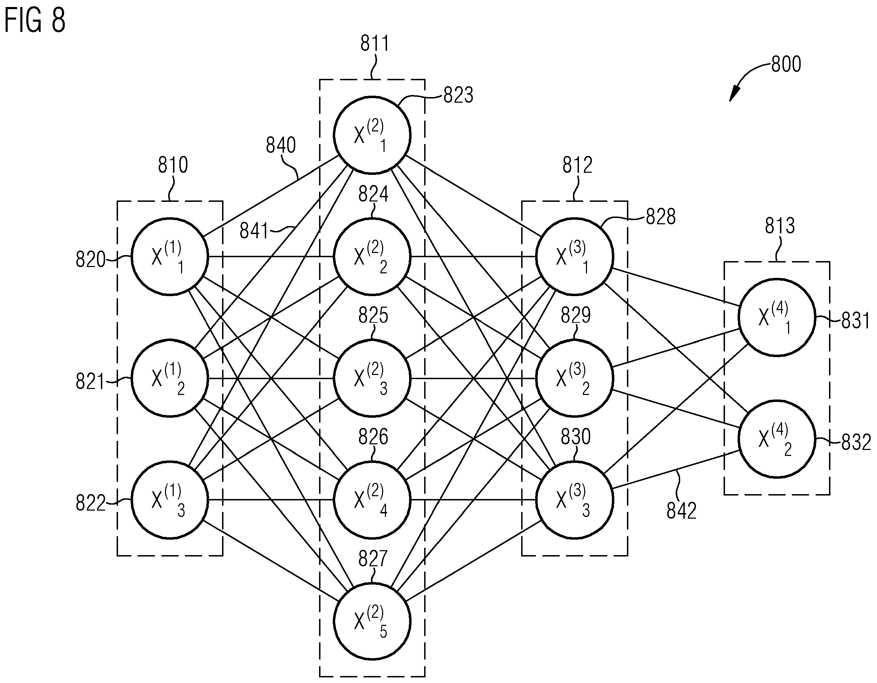

As mentioned above, the second feature extraction module 16 may be an MLP. FIG 8 displays an embodiment of an ANN 800, which is for example designed as an MLP. The ANN 800 comprises nodes 820, ..., 832 and edges 840, ..., 842, wherein each edge 840, ..., 842 is a directed connection from a first node 820, ..., 832 to a second node 820, ..., 832. In general, the first node 820, ..., 832 and the second node 820, ..., 832 are different nodes 820, ..., 832. It is, however, also possible that the first node 820, ..., 832 and the second node 820, ..., 832 are identical. For example, in FIG 8, the edge 840 is a directed connection from the node 820 to the node 823, and the edge 842 is a directed connection from the node 830 to the node 832. An edge 840, ..., 842 from a first node 820, ..., 832 to a second node 820, ..., 832 is also denoted as ingoing edge for the second node 820, ..., 832 and as outgoing edge for the first node 820, ..., 832.

In this example, the nodes 820, ..., 832 of the artificial neural network 800 can be arranged in layers 810, ..., 813, wherein the layers can comprise an intrinsic order introduced by the edges 840, ..., 842 between the nodes 820, ..., 832. In particular, edges 840, ..., 842 can exist only between neighboring layers of nodes. In the displayed example, there is an input layer 810 comprising only nodes 820, ..., 822 without an incoming edge, an output layer 813 comprising only nodes 831, 832 without outgoing edges, and hidden layers 811, 812 in between the input layer 810 and the output layer 813. In general, the number of hidden layers 811, 812 can be chosen arbitrarily. In an MLP, this number is at least one. The number of nodes 820, ..., 822 within the input layer 810 usually relates to the number of input values of the artificial neural network 800, and the number of nodes 831, 832 within the output layer 813 usually relates to the number of output values of the artificial neural network 800.

In particular, a real number can be assigned as a value to every node 820, ..., 832 of the artificial neural network 800. Here, $x^{(n)}_i$ denotes the value of the i-th node 820, ..., 832 of the n-th layer 810, ..., 813. The values of the nodes 820, ..., 822 of the input layer 810 are equivalent to the input values of the artificial neural network 800. The values of the nodes 831, 832 of the output layer 813 are equivalent to the output value of the artificial neural network 800. Furthermore, each edge 840, ..., 842 can comprise a weight being a real number. In particular, the weight is a real number within the interval [-1, 1] or within the interval [0, 1]. Here, $w^{(m,n)}_{i,j}$ denotes the weight of the edge between the i-th node 820, ..., 832 of the m-th layer 810, ..., 813 and the j-th node 820, ..., 832 of the n-th layer 810, ..., 813. Furthermore, the abbreviation $w^{(n)}_{i,j}$ is defined for the weight $w^{(n, n+1)}_{i,j}$. In particular, to calculate the output values of the neural network 800, the input values are propagated through the neural network 800. In particular, the values of the nodes 820, ..., 832 of the (n+1)-th layer 810, ..., 813 can be calculated based on the values of the nodes 820, ..., 832 of the n-th layer 810, ..., 813 by

$$x^{(n+1)}_j = f\left(\sum_i x^{(n)}_i \ w^{(n)}_{i,j}\right).$$

Herein, the function f is denoted as transfer function or activation function. Known transfer functions are step functions, the sigmoid functions, for example the logistic function, the generalized logistic function, the hyperbolic tangent, the arctangent function, the error function, the smoothstep function, or rectifier functions. The transfer function is mainly used for normalization purposes.

In particular, the values are propagated layer-wise through the neural network 800, wherein values of the input layer 810 are given by the input of the neural network 800, wherein values of the first hidden layer 811 can be calculated based on the values of the input layer 810 of the neural network 800, wherein values of the second hidden layer 812 can be calculated based in the values of the first hidden layer 811, and so forth.

In order to set the values $w^{(m,n)}_{i,j}$ for the edges, the neural network 800 has to be trained using training data. In particular, training data comprises training input data and training output data (denoted as $t_i$). For a training step, the neural network 800 is applied to the training input data to generate calculated output data. In particular, the training data and the calculated output data comprise a number of values, said number being equal with the number of nodes of the output layer. In particular, a comparison between the calculated output data and the training data is used to recursively adapt the weights within the neural network 800 (backpropagation algorithm). In particular, the weights are changed according to

$$w_{i,j}'^{(n)} \;=\; w_{i,j}^{(n)} - \gamma\, \delta_j^{(n)}\, x_i^{(n)},$$

wherein $\gamma$ is a predefined learning rate, and the numbers $\delta^{(n)}_j$ can be recursively calculated as

$$\delta_j^{(n)} = \left( \sum_k \delta_k^{(n+1)}\, w_{j,k}^{(n+1)} \right) f'\!\left( x_i^{(n)} w_{i,j}^{(n)} \right)$$

based on $\delta^{(n+1)}_j$, if the (n+1)-th layer is not the output layer 813, and

$$\delta_j^{(n)} = \left( x_j^{(n+1)} - t_j^{(n+1)} \right) f'\!\left( x_i^{(n)} w_{i,j}^{(n)} \right),$$

if the (n+1)-th layer is the output layer 813, wherein f' is the first derivative of the activation function, and $t^{(n+1)}_j$ is the comparison training value for the j-th node of the output layer 813.

As mentioned above, the first feature extraction module 9 may be a CNN. A CNN is an ANN that uses a convolution operation instead of general matrix multiplication in at least one of its layers. These layers are denoted as convolutional layers. In particular, a convolutional layer performs a dot product of one or more convolution kernels with the convolutional layer's input data, wherein the entries of the one or more convolution kernel are parameters or weights that may be adapted by training. In particular, one can use the Frobenius inner product and the ReLU activation function. A convolutional neural network can comprise additional layers, for example pooling layers, fully connected layers, and/or normalization layers.

By using convolutional neural networks, the input can be processed in a very efficient way because a convolution operation based on different kernels can extract various image features so that by adapting the weights of the convolution kernel the relevant image features can be found during training. Furthermore, based on the weight-sharing in the convolutional kernels fewer parameters need to be trained, which prevents overfitting in the training phase and allows to have faster training or more layers in the network, improving the performance of the network.

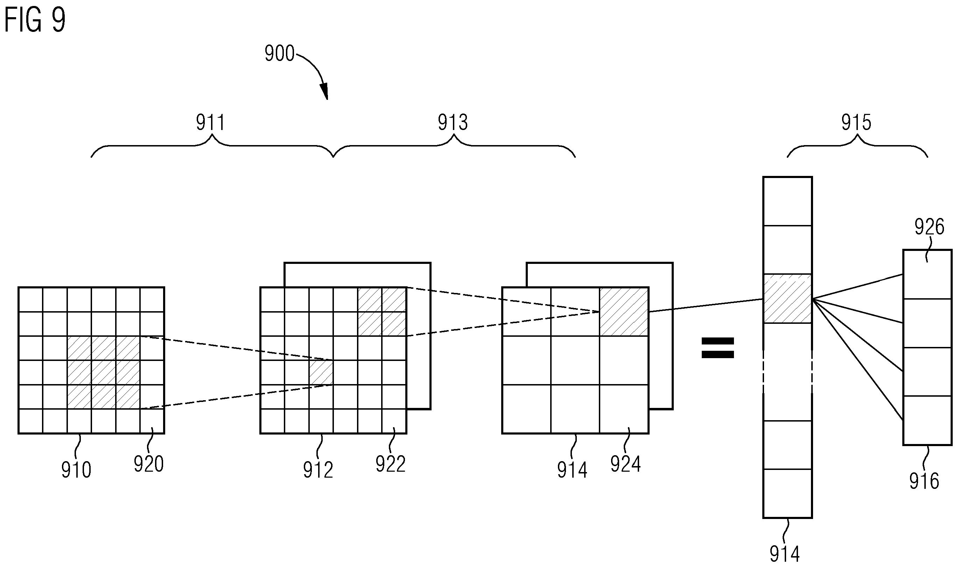

FIG 9 displays an exemplary embodiment of a convolutional neural network 900. In the displayed embodiment, the convolutional neural network 900 comprises an input node layer 910, a convolutional layer 911, a pooling layer 913, a fully connected layer 914 and an output node layer 916, as well as hidden node layers 912, 914. Alternatively, the convolutional neural network 900 can comprise several convolutional layers 911, several pooling layers 913 and/or several fully connected layers 915, as well as other types of layers. The order of the layers can be chosen arbitrarily, usually fully connected layers 915 are used as the last layers before the output layer 916.

In particular, within a convolutional neural network 900 nodes 920, 922, 924 of a node layer 910, 912, 914 can be considered to be arranged as a d-dimensional matrix or as a d-dimensional image. In particular, in the two-dimensional case the value of the node 920, 922, 924 indexed with i and j in the n-th node layer 910, 912, 914 can be denoted as x(n) [i, j]. However, the arrangement of the nodes 920, 922, 924 of one node layer 910, 912, 914 does not have an effect on the calculations executed within the convolutional neural network 900 as such, since these are given solely by the structure and the weights of the edges.

A convolutional layer 911 is a connection layer between an anterior node layer 910 with node values x(n-1) and a posterior node layer 912 with node values x(n). In particular, a convolutional layer 911 is characterized by the structure and the weights of the incoming edges forming a convolution operation based on a certain number of kernels.

In particular, the structure and the weights of the edges of the convolutional layer 911 are chosen such that the values x(n) of the nodes 922 of the posterior node layer 912 are calculated as a convolution x(n) = K * x(n-1) based on the values x(n-1) of the nodes 920 anterior node layer 910, where the convolution * is defined in the two-dimensional case as

$$x^{(n)}[i,j] = \left( K * x^{(n-1)} \right)[i,j] = \sum_{i'} \sum_{j'} K[i',j'] \cdot x^{(n-1)}[i-i', j-j'].$$

Herein, the kernel K is a d-dimensional matrix, in the present example a two-dimensional matrix, which is usually small compared to the number of nodes 920, 922, for example a 3x3 matrix, or a 5x5 matrix. In particular, this implies that the weights of the edges in the convolution layer 911 are not independent, but chosen such that they produce said convolution equation. In particular, for a kernel being a 3x3 matrix, there are only 9 independent weights, each entry of the kernel matrix corresponding to one independent weight, irrespectively of the number of nodes 920, 922 in the anterior node layer 910 and the posterior node layer 912.

In general, convolutional neural networks 900 use node layers 910, 912, 914 with a plurality of channels, in particular, due to the use of a plurality of kernels in convolutional layers 911. In those cases, the node layers can be considered as (d+1)-dimensional matrices, the first dimension indexing the channels. The action of a convolutional layer 911 is then in a two-dimensional example defined as

$$x_b^{(n)}[i,j] = \sum_a (K_{a,b} * x_a^{(n-1)}[i,j] = \sum_a \sum_{i'} \sum_{j'} K_{a,b}[i',j'] \cdot x_a^{(n-1)}[i-i',j-j'],$$

wherein $x_a^{(n)}$ corresponds to the a-th channel of the anterior node layer 910, $x_b^{(n)}$ corresponds to the b-th channel of the posterior node layer 912 and $K_{a,b}$ corresponds to one of the kernels. If a convolutional layer 911 acts on an anterior node layer 910 with A channels and outputs a posterior node layer 912 with B channels, there are A·B independent d-dimensional kernels $K_{a,b}$.

In general, in convolutional neural networks 900 activation functions may be used. In this embodiment, ReLU (rectified linear unit) is used, with R(z) = max(0, z), so that the action of the convolutional layer 911 in the two-dimensional example is

$$x_b^{(n)}[i,j] = R\left(\sum_a (K_{a,b} * x_a^{(n-1)}[i,j]\right)$$
$$= R\left(\sum_a \sum_{i'} \sum_{j'} K_{a,b}[i',j'] \cdot x_a^{(n-1)}[i-i',j-j']\right).$$

It is also possible to use other activation functions, for example ELU (exponential linear unit), LeakyReLU, Sigmoid, Tanh, or Softmax.

In the displayed embodiment, the input layer 910 comprises 36 nodes 920, arranged as a two-dimensional 6x6 matrix. The first hidden node layer 912 comprises 72 nodes 922, arranged as two two-dimensional 6x6 matrices, each of the two matrices being the result of a convolution of the values of the input layer with a 3x3 kernel within the convolutional layer 911. Equivalently, the nodes 922 of the first hidden node layer 912 can be interpreted as arranged as a three-dimensional 2x6x6 matrix, wherein the first dimension corresponds to the channel dimension.

An advantage of using convolutional layers 911 is that spatially local correlation of the input data can be exploited by enforcing a local connectivity pattern between nodes of adjacent layers, in particular by each node being connected to only a small region of the nodes of the preceding layer.

A pooling layer 913 is a connection layer between an anterior node layer 912 with node values x(n-1) and a posterior node layer 914 with node values x(n). In particular, a pooling layer 913 can be characterized by the structure and the weights of the edges and the activation function forming a pooling operation based on a non-linear pooling function f. For example, in the two-dimensional case the values x(n) of the nodes 924 of the posterior node layer 914 can be calculated based on the values x(n-1) of the nodes 922 of the anterior node layer 912 as

$$x_b^{(n)}[i,j] = f\left(x_b^{(n-1)}[id_1, jd_2], ..., x_b^{(n-1)}[(i+1)d_1 - 1, (j+1)d_2 - 1]\right).$$

In other words, by using a pooling layer 913, the number of nodes 922, 924 can be reduced by re-placing a number d1·d2 of neighboring nodes 922 in the anterior node layer 912 with a single node 922 in the posterior node layer 914 being calculated as a function of the values of said number of neighboring nodes. In particular, the pooling function f can be the max-function, the average or the L2-Norm. In particular, for a pooling layer 913 the weights of the incoming edges are fixed and are not modified by training. The advantage of using a pooling layer 913 is that the number of nodes 922, 924 and the number of parameters is reduced. This leads to the amount of computation in the network being reduced and to a control of overfitting.

In the displayed embodiment, the pooling layer 913 is a max-pooling layer, replacing four neighboring nodes with only one node, the value being the maximum of the values of the four neighboring nodes. The max-pooling is applied to each d-dimensional matrix of the previous layer. In this embodiment, the max-pooling is applied to each of the two two-dimensional matrices, reducing the number of nodes from 72 to 18.

In general, the last layers of a convolutional neural network 900 may be fully connected layers 915. A fully connected layer 915 is a connection layer between an anterior node layer 914 and a posterior node layer 916. A fully connected layer 913 can be characterized by the fact that a majority, in particular, all edges between nodes 914 of the anterior node layer 914 and the nodes 916 of the posterior node layer are present, and wherein the weight of each of these edges can be adjusted individually.

In this embodiment, the nodes 924 of the anterior node layer 914 of the fully connected layer 915 are displayed both as two-dimensional matrices, and additionally as non-related nodes, indicated as a line of nodes, wherein the number of nodes was reduced for a better presentability. This operation is also denoted as flattening. In this embodiment, the number of nodes 926 in the posterior node layer 916 of the fully connected layer 915 is smaller than the number of nodes 924 in the anterior node layer 914. Alternatively, the number of nodes 926 can be equal or larger.

Furthermore, in this embodiment the Softmax activation function is used within the fully connected layer 915. By applying the Softmax function, the sum the values of all nodes 926 of the output layer 916 is 1, and all values of all nodes 926 of the output layer 916 are real numbers between 0 and 1. In particular, if using the convolutional neural network 900 for categorizing input data, the values of the output layer 916 can be interpreted as the probability of the input data falling into one of the different categories. In particular, convolutional neural networks 900 can be trained based on the backpropagation algorithm. For preventing overfitting, methods of regularization can be used, for example dropout of nodes 920, ..., 924, stochastic pooling, use of artificial data, weight decay based on the L1 or the L2 norm, or max norm constraints.

In known approaches, users select imaging parameters via a manual, stepwise process. After identifying the broad target area, such as the heart, the imaging system may offer a suite of organ-specific programs. The user then fine-tunes this selection, pinpointing the exact coronary segment or branch for intervention, such as the left anterior descending artery or the right coronary artery, and aligning the program accordingly. This iterative process introduces avenues for oversight and can prolong the procedure, potentially impacting patient outcomes, and using generalized programs leads to less optimal image quality and unnecessary radiation exposure. The invention overcomes said drawbacks.

Independent of the grammatical term usage, individuals with male, female, or other gender identities are included within the term.

## Claims

1. Computer implemented method for automatically determining an operation mode for an X-ray imaging system (1), which comprises an X-ray source (2) and an X-ray detector (3) mounted on a C-arm (4), wherein

   - angulation data (19) defining an angulation state of the C-arm (4) is received;
   - at least one X-ray image (18) depicting an object (6) according to the angulation state is received;

   **characterized in that** the operation mode (22) for the X-ray imaging system (1) is selected as one of two or more predefined operation modes by applying a trained machine learning model, MLM, for classification to input data, the input data comprising the at least one X-ray image (18) and the angulation data (19).

2. Computer implemented method according to claim 1, wherein

   - an information message informing a user about the selected operation mode (22) is generated; and/or
   - the X-ray imaging system (1) is automatically configured according to the selected operation mode (22).

3. Computer implemented method according to claim 2, wherein configuring the X-ray imaging system (1) according to the selected operation mode (22) comprises setting a value of an X-ray exposure time assigned to the selected operation mode (22) and/or setting a value of a frame rate assigned to the selected operation mode (22) and/or setting at least one image processing parameter assigned to the selected operation mode (22) and/or activating or deactivating a function of the X-ray imaging system (1) assigned to the selected operation mode (22).

4. Computer implemented method according to one of the preceding claims, wherein

   - image features are generated by applying a first feature extraction module (9) of the MLM to the at least one X-ray image (18);
   - angulation features are generated by applying a second feature extraction module (16) of the MLM to the angulation data (19);
   - the operation mode is selected depending on the image features and the angulation features.

5. Computer implemented method according to claim 4, wherein

- fused features are generated by fusing the image features with the angulation features; and
- the operation mode is selected depending on the fused features.

6. Computer implemented method according to claim 5, wherein the operation mode is selected by applying at least one fully connected neural network layer (20, 21) of the MLM to the fused features.

7. Computer implemented method according to one of claims 4 to 6, wherein the first feature extraction module (9) comprises a convolutional neural network and/or a residual neural network.

8. Computer implemented method according to one of claims 4 to 7, wherein the second feature extraction module (16) comprises a multi-layer perceptron.

9. Computer implemented method according to one of the preceding claims, wherein the angulation data (19) comprises an angular rotation angle ($\alpha$) and an orbital rotation angle ($\beta$) of the C-arm (4).

10. Computer implemented method according to one of the preceding claims, wherein the two or more predefined operation modes comprise a first operation mode for imaging right coronary arteries and/or a second operation mode for imaging left coronary arteries and/or a third operation mode for imaging a left ventricle.

11. Method for X-ray imaging using an X-ray imaging system (1), which comprises an X-ray source (2) and an X-ray detector (3) mounted on a C-arm (4), wherein

- angulation data (19) defining an angulation state of the C-arm (4) is determined;
- the X-ray imaging system (1) is configured according to at least one preliminary setting;
- at least one X-ray image (18) depicting an object (6) according to the angulation state and according to the at least one preliminary setting is generated by using the X-ray source (2) and the X-ray detector (3);
- a computer implemented method according to one of the preceding claims is carried out;
- the X-ray imaging system (1) is configured according to the selected operation mode (22); and
- a further X-ray image is generated according to the selected operation mode (22) by using the X-ray source (2) and the X-ray detector (3).

12. Computer implemented training method for training an MLM for classification for use in a computer implemented method according to one of claims 1 to 10, wherein

- angulation training data defining a training angulation state of the C-arm (4) is received;
- at least one training image depicting an object (6) according to the training angulation state is received;
- a ground truth annotation for the angulation training data and the at least one training image is received;
- a predicted operation mode for the X-ray imaging system (1) is selected as one of two or more predefined operation modes by applying the MLM to input training data, which comprises the at least one X-ray training image and the training angulation data; and
- the MLM is updated depending on the predicted operation mode and the ground truth annotation.

13. Computer implemented training method according to claim 12, wherein

- training image features are generated by applying a pre-trained first feature extraction module (9) of the MLM to the at least one X-ray training image;
- training angulation features are generated by applying a pre-trained second feature extraction module (16) of the MLM to the angulation training data;
- training fused features are generated by fusing the training image features with the training angulation features;
- the predicted operation mode is selected by applying at least one fully connected neural network layer of the MLM to the fused features; and
- network parameters of the at least one fully connected neural network layer (20, 21) are updated depending on the predicted operation mode and the ground truth annotation.

14. Data processing apparatus comprising

- at least one computing unit (5), which is adapted to carry out a computer implemented method according to one of claims 1 to 10; and/or

- at least one further computing unit, which is adapted to carry out a computer implemented training method according to one of claims 12 or 13.

**15.** X-ray imaging system (1) comprising an X-ray source (2) and an X-ray detector (3) mounted on a C-arm (4) and at least one computing unit (5), which is adapted to carry out a computer implemented method according to one of claims 1 to 10.

**16.** Computer program product comprising

- first instructions, which, when executed by a data processing apparatus, cause the data processing apparatus to carry out a computer implemented method according to one of claims 1 to 10; and/or
- second instructions, which, when executed by a data processing apparatus, cause the data processing apparatus to carry out a computer implemented training method according to one of claims 12 or 13; and/or
- third instructions, which, when executed by an X-ray imaging system (1) according to claim 15, cause the X-ray imaging system (1) to carry out a method according to claim 11.


**Patentansprüche**

**1.** Computerimplementiertes Verfahren zum automatischen Bestimmen eines Betriebsmodus für ein Röntgenbildgebungssystem (1), das eine Röntgenquelle (2) und einen Röntgendetektor (3) umfasst, die auf einem C-Arm (4) montiert sind, wobei

- Angulationsdaten (19) empfangen werden, die einen Angulationszustand des C-Arms (4) definieren;
- Mindestens ein Röntgenbild (18), das ein Objekt (6) gemäß dem Angulationszustand darstellt, empfangen wird;

**dadurch gekennzeichnet, dass** der Betriebsmodus (22) für das Röntgenbildgebungssystem (1) durch Anwenden eines trainierten Maschinenlernmodells (MLM) zur Klassifizierung auf Eingabedaten als einer von zwei oder mehr vordefinierten Betriebsmodi ausgewählt wird, wobei die Eingabedaten das mindestens eine Röntgenbild (18) und die Angulationsdaten (19) umfassen.

**2.** Computerimplementiertes Verfahren nach Anspruch 1, wobei

- eine Informationsnachricht erzeugt wird, die einen Benutzer über den ausgewählten Betriebsmodus (22) informiert; und/oder
- das Röntgenbildgebungssystem (1) automatisch entsprechend dem ausgewählten Betriebsmodus (22) konfiguriert wird.

**3.** Computerimplementiertes Verfahren nach Anspruch 2, wobei das Konfigurieren des Röntgenbildgebungssystems (1) gemäß dem ausgewählten Betriebsmodus (22) das Einstellen eines Wertes einer Röntgenbelichtungszeit, die dem ausgewählten Betriebsmodus (22) zugewiesen ist, und/oder das Einstellen eines Wertes einer Bildrate, die dem ausgewählten Betriebsmodus (22) zugewiesen ist, und/oder das Einstellen mindestens eines Bildverarbeitungsparameters, der dem ausgewählten Betriebsmodus (22) zugewiesen ist, und/oder das Aktivieren oder Deaktivieren einer Funktion des Röntgenbildgebungssystems (1), das dem ausgewählten Betriebsmodus (22) zugewiesen ist, umfasst.

**4.** Computerimplementiertes Verfahren nach einem der vorhergehenden Ansprüche, wobei

- Bildmerkmale durch Anwenden eines ersten Merkmalsextraktionsmoduls (9) des MLM auf das mindestens eine Röntgenbild (18) erzeugt werden;
- Angulationsmerkmale durch Anwenden eines zweiten Merkmalsextraktionsmoduls (16) des MLM auf die Angulationsdaten (19) erzeugt werden;
- der Betriebsmodus abhängig von den Bildmerkmalen und den Angulationsmerkmalen ausgewählt wird.

**5.** Computerimplementiertes Verfahren nach Anspruch 4, wobei

- fusionierte Merkmale durch Verschmelzen der Bildmerkmale mit den Angulationsmerkmalen erzeugt werden; und

- der Betriebsmodus abhängig von den fusionierten Funktionen ausgewählt wird.

6. Computerimplementiertes Verfahren nach Anspruch 5, wobei der Betriebsmodus durch Anwenden mindestens einer vollständig verbundenen neuronalen Netzwerkschicht (20, 21) des MLM auf die fusionierten Merkmale ausgewählt wird.

7. Computerimplementiertes Verfahren nach einem der Ansprüche 4 bis 6, wobei das erste Merkmalsextraktionsmodul (9) ein neuronales Faltungsnetzwerk und/oder ein neuronales Restnetzwerk umfasst.

8. Computerimplementiertes Verfahren nach einem der Ansprüche 4 bis 7, wobei das zweite Merkmalsextraktionsmodul (16) ein mehrschichtiges Perzeptron umfasst.

9. Computerimplementiertes Verfahren nach einem der vorhergehenden Ansprüche, wobei die Angulationsdaten (19) einen Winkeldrehwinkel ($\alpha$) und einen Orbitaldrehwinkel ($\beta$) des C-Arms (4) umfassen.

10. Computerimplementiertes Verfahren nach einem der vorhergehenden Ansprüche, wobei die zwei oder mehr vordefinierten Betriebsmodi einen ersten Betriebsmodus zum Abbilden rechter Koronararterien und/oder einen zweiten Betriebsmodus zum Abbilden linker Koronararterien und/oder einen dritten Betriebsmodus zum Abbilden einer linken Herzkammer umfassen.

11. Verfahren zur Röntgenbildgebung unter Verwendung eines Röntgenbildgebungssystems (1), das eine Röntgenquelle (2) und einen Röntgendetektor (3) umfasst, die auf einem C-Arm (4) montiert sind, wobei

- Angulationsdaten (19), die einen Angulationszustand des C-Arms (4) definieren, bestimmt werden;
- das Röntgenbildgebungssystem (1) gemäß mindestens einer vorläufigen Einstellung konfiguriert ist;
- mindestens ein Röntgenbild (18), das ein Objekt (6) gemäß dem Angulationszustand und gemäß der mindestens einen Voreinstellung darstellt, unter Verwendung der Röntgenquelle (2) und des Röntgendetektors (3) erzeugt wird;
- ein computerimplementiertes Verfahren nach einem der vorhergehenden Ansprüche durchgeführt wird;
- das Röntgenbildgebungssystem (1) gemäß dem ausgewählten Betriebsmodus (22) konfiguriert ist; und
- ein weiteres Röntgenbild gemäß dem gewählten Betriebsmodus (22) unter Verwendung der Röntgenquelle (2) und des Röntgendetektors (3) erzeugt wird.

12. Computerimplementiertes Trainingsverfahren zum Trainieren eines MLM zur Klassifizierung zur Verwendung in einem computerimplementierten Verfahren nach einem der Ansprüche 1 bis 10, wobei

- Angulationstrainingsdaten, die einen Trainingsangulationszustand des C-Arms (4) definieren, empfangen werden;
- mindestens ein Trainingsbild, das ein Objekt (6) gemäß dem Trainingsangulationszustand darstellt, empfangen wird;
- eine Grundwahrheitsannotation für die Angulationstrainingsdaten und das mindestens eine Trainingsbild empfangen wird;
- ein vorhergesagter Betriebsmodus für das Röntgenbildgebungssystem (1) als einer von zwei oder mehr vordefinierten Betriebsmodi durch Anwenden des MLM auf eingegebene Trainingsdaten ausgewählt wird, die das mindestens eine Röntgentrainingsbild und die Trainingsangulationsdaten umfassen; und
- das MLM in Abhängigkeit vom vorhergesagten Betriebsmodus und der Grundwahrheitsanmerkung aktualisiert wird.

13. Computerimplementiertes Trainingsverfahren nach Anspruch 12, wobei

- Trainingsbildmerkmale durch Anwenden eines vortrainierten ersten Merkmalsextraktionsmoduls (9) des MLM auf das mindestens eine Röntgentrainingsbild erzeugt werden;
- Trainingsangulationsmerkmale durch Anwenden eines vortrainierten zweiten Merkmalsextraktionsmoduls (16) des MLM auf die Angulationstrainingsdaten erzeugt werden;
- durch Verschmelzen der Trainingsbildmerkmale mit den Trainingsangulationsmerkmalen zusammengesetzte Trainingsmerkmale erzeugt werden;
- der vorhergesagte Betriebsmodus durch Anwenden mindestens einer vollständig verbundenen neuronalen Netzwerkschicht des MLM auf die fusionierten Merkmale ausgewählt wird; und

- Netzwerkparameter der mindestens einen vollständig verbundenen neuronalen Netzwerkschicht (20, 21) in Abhängigkeit von dem vorhergesagten Betriebsmodus und der Grundwahrheitsannotation aktualisiert werden.

14. Datenverarbeitungsvorrichtung, die Folgendes umfasst:

- mindestens eine Recheneinheit (5), die geeignet ist, ein computerimplementiertes Verfahren nach einem der Ansprüche 1 bis 10 auszuführen; und/oder
- mindestens eine weitere Recheneinheit, die geeignet ist, ein computerimplementiertes Trainingsverfahren nach einem der Ansprüche 12 oder 13 durchzuführen.

15. Röntgenbildgebungssystem (1), umfassend eine Röntgenquelle (2) und einen Röntgendetektor (3), die auf einem C-Arm (4) montiert sind, und mindestens eine Recheneinheit (5), die dazu ausgelegt ist, ein computerimplementiertes Verfahren nach einem der Ansprüche 1 bis 10 durchzuführen.

16. Computerprogrammprodukt, das Folgendes umfasst:

- erste Anweisungen, die bei Ausführung durch eine Datenverarbeitungsvorrichtung die Datenverarbeitungsvorrichtung veranlassen, ein computerimplementiertes Verfahren nach einem der Ansprüche 1 bis 10 auszuführen; und/oder
- zweite Anweisungen, die bei Ausführung durch eine Datenverarbeitungsvorrichtung die Datenverarbeitungsvorrichtung veranlassen, ein computerimplementiertes Verfahren nach einem der Ansprüche 12 oder 13 auszuführen; und/oder
- dritte Anweisungen, die bei Ausführung durch ein Röntgenbildgebungssystem (1) nach Anspruch 15 bewirken, dass das Röntgenbildgebungssystem (1) ein Verfahren nach Anspruch 11 ausführt.

## Revendications

1. Procédé mis en œuvre par ordinateur de détermination de manière automatique d'un mode de fonctionnement d'un système (1) d'imagerie aux rayons X, qui comprend une source (2) de rayons X et un détecteur (3) de rayons X monté sur un bras (4) en C, dans lequel

- on reçoit une donnée (19) d'angulation définissant un état d'angulation du bras (4) en C ;
- on reçoit au moins une image (18) aux rayons X décrivant un objet (6) conformément à l'état d'angulation ;

**caractérisé en ce que** le mode (22) de fonctionnement du système (1) d'imagerie aux rayons X est choisi comme l'un de deux ou plusieurs modes de fonctionnement définis à l'avance en appliquant un modèle d'apprentissage par machine, MLM, entraîné à la classification d'une donnée d'entrée, la donnée d'entrée comprenant la au moins une image (18) aux rayons X et la donnée (19) d'angulation.

2. Procédé mis en œuvre par ordinateur suivant la revendication 1, dans lequel

- on crée un message d'information informant un utilisateur du mode (22) de fonctionnement choisi ; et/ou
- on configure automatiquement le système (1) d'imagerie aux rayons X conformément au mode (22) de fonctionnement choisi.

3. Procédé mis en œuvre par ordinateur suivant la revendication 2, dans lequel configurer le système (1) d'imagerie aux rayons X conformément au mode (22) de fonctionnement choisi comprend fixer une valeur d'un temps d'exposition aux rayons X affecté au mode (22) de fonctionnement choisi et/ou fixer une valeur d'un taux de trame affecté au mode (22) de fonctionnement choisi et/ou fixer au moins un paramètre de traitement de l'image affecté au mode (22) de fonctionnement choisi et/ou activer ou désactiver une fonction du système (1) d'imagerie par rayons X affectée au mode (22) de fonctionnement choisi.

4. Procédé mis en œuvre par ordinateur suivant l'une des revendications précédentes, dans lequel

- on crée des caractéristiques d'image en appliquant un premier module (9) d'extraction de caractéristiques du MLM à la au moins une image (18) aux rayons X ;
- on crée des caractéristiques d'angulation en appliquant un deuxième module (16) d'extraction de caractéristiques du MLM à la donnée (19) d'angulation ;
- on choisit le mode de fonctionnement en fonction des caractéristiques d'image et des caractéristiques d'angulation.

5. Procédé mis en œuvre par ordinateur suivant la revendication 4, dans lequel

    - on crée des caractéristiques fusionnées en fusionnant les caractéristiques d'image avec les caractéristiques d'angulation ; et
    - on sélectionne le mode de fonctionnement en fonction des caractéristiques fusionnées.

6. Procédé mis en œuvre par ordinateur suivant la revendication 5, dans lequel on choisit le mode de fonctionnement en appliquant au moins une couche (20, 21) de réseau neuronal connectée entièrement du MLM aux caractéristiques fusionnées.

7. Procédé mis en œuvre par ordinateur suivant l'une des revendications 4 à 6, dans lequel le premier module (9) d'extraction de caractéristiques comprend un réseau neuronal de convolution et/ou un réseau neuronal résiduel.

8. Procédé mis en œuvre par ordinateur suivant l'une des revendications 4 à 7, dans lequel le deuxième module (16) d'extraction de caractéristiques comprend un perceptron à couches multiples.

9. Procédé mis en œuvre par ordinateur suivant l'une des revendications précédentes, dans lequel la donnée (19) d'angulation comprend un angle ($\alpha$) angulaire de rotation et un angle ($\beta$) orbital de rotation du bras (4) en C.

10. Procédé mis en œuvre par ordinateur suivant l'une des revendications précédentes, dans lequel les deux ou plusieurs modes de fonctionnement définis à l'avance comprennent un premier mode de fonctionnement d'imagerie des artères coronaires droites et/ou un deuxième mode de fonctionnement d'imagerie des artères coronaires gauches et/ou un troisième mode de fonctionnement d'imagerie du ventricule gauche.

11. Procédé d'imagerie aux rayons X en utilisant un système (1) d'imagerie aux rayons X, qui comprend une source (2) de rayons X et un détecteur (3) de rayons X monté sur un bras (4) en C, dans lequel

    - on détermine une donnée (19) d'angulation définissant un état d'angulation du bras (4) en C ;
    - on configure le système (1) d'imagerie aux rayons X conformément à au moins un réglage préliminaire ;
    - en utilisant la source (2) de rayons X et le détecteur (3) de rayons X, on crée au moins une image (18) aux rayons X décrivant un objet (6) conformément à l'état d'angulation et conformément au au moins un réglage préliminaire ;
    - on effectue un procédé mis en œuvre par ordinateur suivant l'une des revendications précédentes ;
    - on configure le système (1) d'imagerie aux rayons X suivant le mode (22) de fonctionnement choisi ; et
    - en utilisant la source (2) de rayons X et le détecteur (3) de rayons X, on crée une autre image aux rayons X suivant le mode (22) de fonctionnement choisi.

12. Procédé d'entraînement mis en œuvre par ordinateur pour entraîner un MLM de classification à utiliser dans un procédé mis en œuvre par ordinateur suivant l'une des revendications 1 à 10, dans lequel

    - on reçoit une donnée d'entraînement d'angulation définissant un état d'angulation d'entraînement du bras (4) en C ;
    - on reçoit au moins une image d'entraînement décrivant un objet (6) conformément à l'état d'angulation d'entraînement ;
    - on reçoit une annotation de vérité de terrain pour la donnée d'entraînement d'angulation et la au moins une image d'entraînement ;
    - on choisit un mode de fonctionnement prédit du système (1) d'imagerie aux rayons X comme l'un de deux ou plusieurs modes de fonctionnement définis à l'avance en appliquant le MLM à une donnée d'entraînement d'entrée, qui comprend la au moins une image d'entraînement aux rayons X et la donnée d'angulation d'entraînement ; et
    - on met à jour le MLM en fonction du mode de fonctionnement prédit et de l'annotation de vérité de terrain.

13. Procédé d'entraînement mis en œuvre par ordinateur suivant la revendication 12, dans lequel

    - on crée des caractéristiques d'image d'entraînement en appliquant un premier module (9) pré-entraîné d'extraction de caractéristiques du MLM à la au moins une image d'entraînement aux rayons X ;
- on crée les caractéristiques d'angulation d'entraînement en appliquant un deuxième module (16) pré-entraîné d'extraction de caractéristiques du MLM à la donnée d'entraînement d'angulation ;
- on crée des caractéristiques fusionnées d'entraînement en fusionnant les caractéristiques d'image d'entraînement avec les caractéristiques d'angulation d'entraînement ;
- on choisit le mode de fonctionnement prédit en appliquant au moins une couche de réseau neuronal connectée entièrement du MLM aux caractéristiques fusionnées ; et
- on met à jour des paramètres de réseau de la au moins une couche (20, 21) de réseau neuronal connectée entièrement en fonction du mode de fonctionnement prédit et de l'annotation de vérité de terrain.

**14.** Installation de traitement de données comprenant

- au moins une unité (5) informatique, qui est propre à effectuer un procédé de mise en œuvre par ordinateur suivant l'une des revendications 1 à 10 ; et/ou
- au moins une autre unité informatique, qui est propre à effectuer un procédé d'entraînement mis en œuvre par ordinateur suivant l'une des revendications 12 ou 13.

**15.** Système (1) d'imagerie aux rayons X comprenant une source (2) de rayons X et un détecteur (3) de rayons X monté sur un bras (4) en C et au moins une unité (5) informatique, qui est propre à effectuer un procédé mis en œuvre par ordinateur suivant l'une des revendications 1 à 10.

**16.** Produit de programme d'ordinateur comprenant

- des premières instructions, qui, lorsqu'elles sont exécutées par une installation de traitement de données, font que l'installation de traitement de données exécute un procédé mis en œuvre par ordinateur suivant l'une des revendications 1 à 10 ; et/ou
- des deuxièmes instructions, qui, lorsqu'elles sont exécutées par une installation de traitement de données, font que l'installation de traitement de données exécute un procédé d'entraînement mis en œuvre par ordinateur suivant l'une des revendications 12 ou 13 ; et/ou
- des troisièmes instructions, qui, lorsqu'elles sont exécutées par un système (1) d'imagerie aux rayons X suivant la revendication 15, font que le système (1) d'imagerie aux rayons X exécute un procédé suivant la revendication 11.

FIG 1

FIG 2

```
┌──────────┐
│   200    │
└────┬─────┘
     │
     ▼
┌──────────┐
│   210    │
└────┬─────┘
     │
     ▼
┌──────────┐
│   220    │
└────┬─────┘
     │
     ▼
┌──────────┐
│   230    │
└────┬─────┘
     │
     ▼
┌──────────┐
│   240    │
└────┬─────┘
     │
     ▼
┌──────────┐
│   250    │
└──────────┘
```

FIG 3

```
┌──────────┐         ┌──────────┐
│   300    │         │   310    │
└────┬─────┘         └────┬─────┘
     │                    │
     ▼                    ▼
   ┌────────────────────────┐
   │          320           │◄───┐
   └───────────┬────────────┘    │
               │                 │
               ▼                 │
   ┌────────────────────────┐    │
   │          330           │    │
   └───────────┬────────────┘    │
               │                 │
               ▼                 │
   ┌────────────────────────┐    │
   │          340           │    │
   └───────────┬────────────┘    │
               │                 │
               ▼                 │
   ┌────────────────────────┐    │
   │          350           │    │
   └───────────┬────────────┘    │
               │                 │
               ▼                 │
   ┌────────────────────────┐    │
   │          360           │    │
   └───────────┬────────────┘    │
               │                 │
               ▼                 │
             ◇ ──────────────────┘
          370  ──►  ◯ ── 380
```

FIG 4

FIG 5

FIG 6

FIG 7

FIG 8

FIG 9

EP 4 585 152 B1

**EP 4 585 152 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20230020252 A1

**Non-patent literature cited in the description**

- **K. HE et al.** Deep Residual Learning for Image Recognition. *Proceedings of the IEEE Conference on Computer Vision and Pattern Recognition (CVPR)*, 2016, 770-778